# EUROPEAN PATENT SPECIFICATION

(11) **EP 2 209 834 B1**
(45) Date of publication and mention of the grant of the patent: **08.06.2011**
(21) Application number: 08848988.5
(22) Date of filing: 12.11.2008
(51) Int. Cl.: C08G 65/334

(54) **HETEROBIFUNCTIONAL POLYETHYLENE GLYCOL REAGENTS**
HETEROBIFUNKTIONELLE POLYETHYLENGLYCOL-REAGENZIEN
RÉACTIFS DE TYPE POLYÉTHYLÈNE GLYCOL HÉTÉROBIFONCTIONNEL

(30) Priority: 12.11.2007 US 2853
(43) Date of publication of application: 28.07.2010
(73) Proprietor: Intradigm Corporation, Palo Alto, CA 94303 (US)
(72) Inventor: LEVY, Daniel E., San Mateo California 94402 (US); ZALIPSKY, Samuel, Redwood City California 94061 (US); CHAMOW, Steven M., San Mateo California 94403 (US)
(74) Representative: Vossius & Partner
(86) International application number: PCT/US2008/012782
(87) International publication number: WO 2009/064459

(56) References cited:
- WO-A-97/41897
- US-A1- 2005 209 416
- THOMPSON ET AL: "Synthesis and applications of heterobifunctional poly(ethylene oxide) oligomers" POLYMER, ELSEVIER SCIENCE PUBLISHERS B.V, GB, vol. 49, no. 2, 24 October 2007 (2007-10-24), pages 345-373, XP022422554 ISSN: 0032-3861
- LI JING ET AL: "Synthesis of polyethylene glycol (PEG) derivatives and PEGylated-peptide biopolymer conjugates" BIOMACROMOLECULES, ACS, WASHINGTON, DC, US, vol. 4, no. 4, 17 May 2003 (2003-05-17), pages 1055-1067, XP002328259 ISSN: 1525-7797

## Description

### Field of the Invention

The invention relates to heterobifunctional polyethylene glycol reagents, methods of producing them and methods of using them.

### Background of the Invention

Polyethyene glycol (PEG) is an inert, non-toxic water-soluble polymer. As a result of these and other desirable properties, derivatives of PEG have found use as reagents in a variety of pharmaceutical, biomedical and biotechnical applications.

However, unmodified PEG itself is not useful as a reagent, due to limited reactivity of the terminal hydroxyl groups. Thus, to make a useful PEG reagent, one of the terminal hydroxyl groups must be modified to a more reactive group. According to prior art methods, new functionality was applied to one of the terminal hydroxy groups by first blocking the other terminal hydroxyl group as an alkyl ether or another non-reactive functionality and then converting the unblocked terminal hydroxy group to an electrophilic center, such as an activated carboxylate. Alternatively, functionality was applied to one terminal hydroxyl group and the mono-functionalized PEG was isolated from a mixture of unmodified PEG, mono-functionalized PEG and bi-functionalized PEG. (Morpurgo et al., Bioconjugate Chem., 7, 363-368, (1996); Zalipsky et al., J. Bioactive and Compatible Polymers, 5, 227-231, (1990); Snow et al., U.S. Patent No. 5,414,135; Shadle et al., U.S. Patent No. 4,847,325).

Bifunctional PEG reagents contain reactive groups on both ends of the PEG molecule. These bifunctional reagents may contain the same reactive group on both terminal ends of the PEG (i.e., homobifunctional PEG reagents) or different groups (i.e., hetero-bifunctional PEG reagents). Heterobifunctional PEG reagents provide advantages over homobifunctional PEG reagents in that each functional group of the heterobifunctional PEG reagent can form a covalent attachment with a different molecule on each terminus. In such a reaction, the two separate molecules become linked by the PEG polymer. However, synthesizing bifunctional reagents can be complicated, particularly when different functionalities are desired on each end of the PEG molecule (i.e., heterobifunctional reagents).

Thus, there remains a significant need to develop new heterobifunctional PEG reagents, and methods for producing such reagents.

### Summary of the Invention

The present invention provides novel classes of heterobifunctional PEG reagents. Heterobifunctional PEG reagents of the present invention have the above-mentioned and other advantages over the prior art. These reagents of the present invention comprise new and useful combinations of functional groups and can react with one or more suitable target molecules. Upon such a reaction, the PEG becomes covalently attached to one or more target molecule(s).

The heterobifunctional PEG reagents of the present invention provide two functional groups that can have different relative reactivities. In some embodiments, one functional group may be more reactive than the other. In other embodiments, one functional group may prefer, or even be selective, for a particular target molecule. In other embodiments, one functional group may selectively form a covalent bond with a target under certain reaction conditions.

The difference in reactivity between the functional groups facilitates the attachment to two different molecules because the first functional group can react and form a bond with a first target molecule, even in the presence of the second functional group. Subsequently, the second functional group, having a different relative reactivity, can then react and form a bond with a second target molecule.

In one aspect, the present invention provides a reagent comprising a compound of the formula (I): 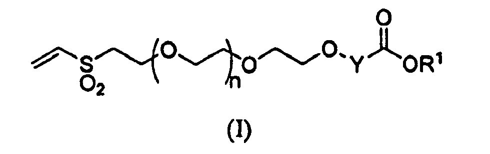 wherein:
Y is selected from the group consisting of C₁-C₆ alkylene, C₂-C₆ alkenylene, and C₂-C₆ alkynylene, wherein Y is optionally substituted with one or more substituents R²;
R² is independently selected from the group consisting of branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂,
CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
R¹ is C₆-C₁₄ aryl, or C₅-C₁₄ heteroaryl containing one or more heteroatoms selected from the group consisting of N, N(R⁷), O, S, and S(O)ₘ, wherein m is 1 or 2, and wherein R¹ is optionally substituted with one or more substituents R⁸;
R⁷ is selected from the group consisting of H, branched or straight-chain C₁-C₆ alkyl, branched or straight-chain C₂-C₆ alkenyl, branched or straight-chain C₂-C₆ alkynyl wherein one or more hydrogens in the alkyl, alkenyl or alkynyl chain may be replaced by one or more fluorines,
R⁸ is independently selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl)₂, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines; and
n is an integer from 1 to 1,500.

In certain embodiments of compound (I), n is preferably an integer from 50 to 250. In certain embodiments of compound (I), R¹ is preferably phenyl, pyridyl, pyrimidinyl, or naphthyl, in which the ring is optionally substituted by one or more substituents R⁸. In certain embodiments of compound (I), R¹ is preferably phenyl and R⁸ is nitro.

In another aspect, the present invention provides a reagent comprising a compound of the formula (II): 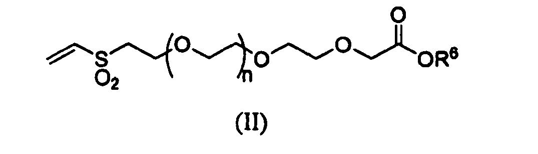 wherein R⁶ is H, branched or straight-chain C₁-C₆ alkyl, branched or straight-chain C₂-C₆ alkenyl, branched or straight-chain C₂-C₆ alkynyl; and
n is an integer from 1 to 1,500.

In certain embodiments of compound (II), n is preferably an integer from 50 to 250. In certain embodiments, R⁶ is preferably H or methyl.

In another aspect, the present invention provides a method for producing a heterobifunctional PEG compound of formula (XIV): 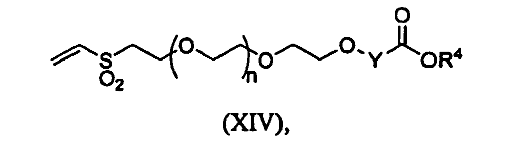 comprising the steps of:
(a) reacting a compound of formula (III) with divinyl sulfone: 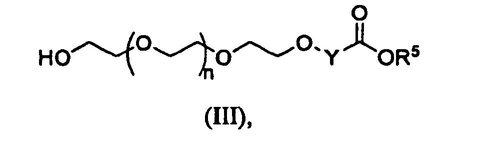 to form a compound of formula (XV): 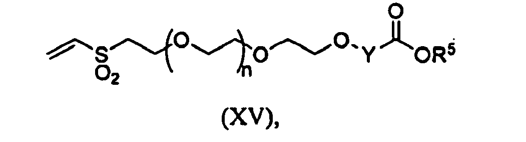 and (b) reacting the compound of formula (XV) with HO-R⁴ in the presence of an ester coupling agent to form the compound of formula (XIV),
wherein:
Y is selected from the group consisting of C₁-C₆ alkylene, C₂-C₆ alkenylene, and C₂-C₆ alkynylene, wherein Y is optionally substituted with one or more substituents R²;
R² is independently selected from the group consisting of branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
R⁵ is H;
R⁴ is C₆-C₁₄ aryl or C₅-C₁₄ heteroaryl containing one or more heteroatoms selected from N, N(R⁷), O, S, S(O)ₘ, wherein m is 1 or 2, wherein R⁴ is optionally substituted with one or more substituents R⁸;
R⁷ is selected from the group consisting of H, branched or straight-chain C₁-C₆ alkyl, branched or straight-chain C₂-C₆ alkenyl, branched or straight-chain C₂-C₆ alkynyl, wherein one or more hydrogens in the alkyl, alkenyl or alkynyl chain may be replaced by one or more fluorines;
R⁸ is selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl)₂, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines; and
n is an integer from 1 to 1,500.

In certain embodiments, HO-R⁴ is *p*-nitrophenol and n is an integer from 50 to 250.

The above method advantageously provides an efficient method for reacting divinyl sulfone with compound (III) even in the presence of a free acid.

In another aspect, the present invention provides a method for producing a heterobifunctional PEG compound of formula (XIV): 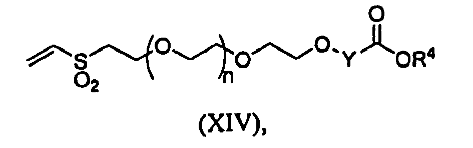 comprising the step of:
(a) reacting a compound of formula (XV) 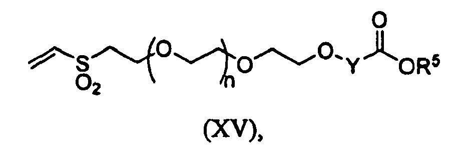 with R¹⁴-R⁴ in the presence of an ester coupling agent to form the compound of formula (XIV),
wherein Y, R², R⁵, R⁴ and n are defined as above, and
R¹⁴ is selected from the group consisting of HO-, CF₃C(O)-O-, CH₃S(O)₂-O-, CF₃S(O)₂-O-, CH₃(C₆H₄)S(O)₂-O-, Cl, Br, and I.

In certain embodiments, R¹⁴ is CF₃C(O)-O-, R⁴ is phenyl and R⁸ is nitro.

In another aspect, the present invention provides a method for producing a compound of formula (VIII): 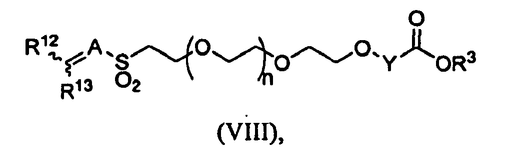 comprising the steps of:
(a) reacting a compound of formula (IV) 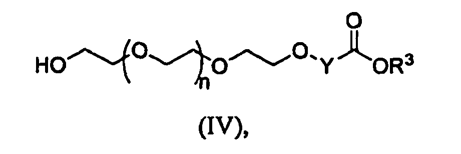 with an activating group to form a compound of formula (IVa): 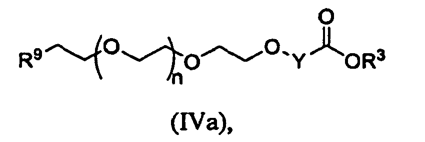
(b) reacting the compound of formula (IVa) with a compound of formula (V): 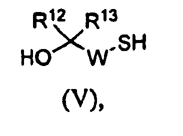 to form a compound of formula (VI): 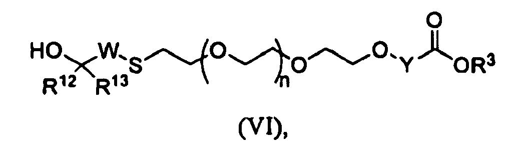
(c) oxidizing the compound of formula (VI) to form a compound of formula (VII): 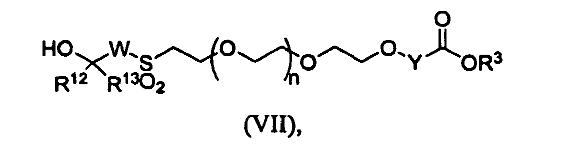 and (d) dehydrating the compound of formula (VII) to form a compound of formula (VIII): 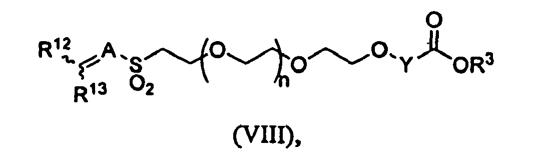
wherein:
W is C₁-C₆ alkylene, wherein W is optionally substituted with one or more substituents R¹¹;
A is C₁-C₆ 1-alkanyl-ylidene, wherein A is optionally substituted with one or more substituents R¹¹;
R¹¹ is independently selected from the group consisting of H, branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
Y is selected from the group consisting of C₁-C₆ alkylene, C₂-C₆ alkenylene, and C₂-C₆ alkynylene, wherein Y is optionally substituted with one or more substituents R²; R² is independently selected from the group consisting of branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
R³ is H, branched or straight-chain C₁-C₆ alkyl, branched or straight-chain C₂-C₆ alkenyl, branched or straight-chain C₂-C₆ alkynyl, C₆-C₁₄ aryl or C₅-C₁₄ heteroaryl containing one or more heteroatoms selected from N, N(R⁷), O, S, S(O)ₘ, wherein m is 1 or 2, provided that when R³ is not H, R³ is optionally substituted with one or more substituents R⁸;
R⁷ is selected from the group consisting of H, branched or straight-chain C₁-C₆ alkyl, branched or straight-chain C₂-C₆ alkenyl, and branched or straight-chain C₂-C₆ alkynyl, wherein one or more hydrogens in the alkyl, alkenyl or alkynyl chain may be replaced by one or more fluorines;
R⁸ is selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl)₂, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
R⁹ is selected from the group consisting of CH₃S(O)₂-O-, CF₃S(O)₂-O-, CH₃(C₆H₄)S(O)₂-O-, Cl, Br, and I;
R¹² is selected from the group consisting of H, branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
R¹³ is selected from the group consisting of H, branched or straight-chain C₁-C₆ alkyl, branched or straight-chain C₂-C₆ alkenyl, branched or straight-chain C₂-C₆ alkynyl, hydroxy, C₁-C₆alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
and n is an integer from 1 to 1,500.

The above method advantageously provides an efficient method for forming a vinyl sulfone adduct of the compound of formula (IV) by an *in situ* formation of the vinyl sulfone.

In a further aspect, the present invention provides a method for producing a compound of formula (X), 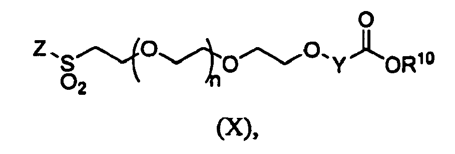 comprising the step of:
(a) reacting a compound of formula (IX): 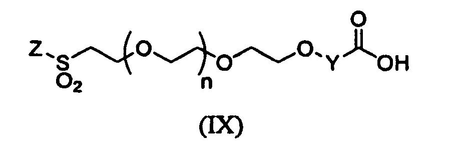 with an activating reagent to form the compound of formula (X),
   wherein:
   the activating reagent comprises R¹⁰-R¹⁴,
   R¹⁰ is a branched or straight-chain C₁-C₆ alkyl, branched or straight-chain C₂-C₆ alkenyl, branched or straight-chain C₂-C₆ alkynyl, C₆-C₁₄ aryl or C₅-C₁₄ heteroaryl containing one or more heteroatoms selected from the group consisting of N, N(R⁷), O, S, and S(O)ₘ, wherein m is 1 or 2, wherein R¹⁰ is optionally substituted with one or more substituents R⁸;
   R⁷ is selected from the group consisting of H, branched or straight-chain C₁-C₆ alkyl, branched or straight-chain C₂-C₆ alkenyl, branched or straight-chain C₂-C₆ alkynyl, wherein one or more hydrogens in the alkyl, alkenyl or alkynyl chain may be replaced by one or more fluorines;
   R⁸ is independently selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl)₂, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   R¹⁴ is selected from the group consisting of HO-, CF₃C(O)-O-, CH₃S(O)₂-O-, CF₃S(O)₂-O-, CH₃(C₆H₄)S(O)₂-O-, Cl, Br, and I;
   Z is selected from the group consisting of-CH₂CH₂Cl, -CH₂CH₂Br, -CH₂CH₂I, -CH₂CH₂OS(O)₂CH_{3,} -CH₂CH₂OS(O)₂CF₃, -CH₂CH₂OS(O)₂ (C₆H₄)CH₃, and -CH=CH₂;
   Y is selected from the group consisting of C₁-C₆ alkylene, C₂-C₆ alkenylene, and C₂-C₆ alkynylene, wherein Y is optionally substituted with one or more substituents R², R² is independently selected from the group consisting of branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   and n is an integer from 1 to 1,500.

In another aspect, the present invention provides methods of using a heterobifunctional PEG to form a vector of formula (XVI): 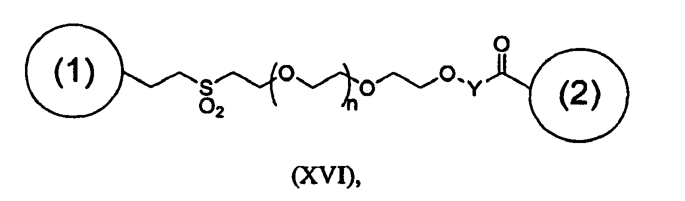 comprising the step of mixing a heterobifunctional PEG of the present invention with (**1**) and (**2**), wherein (**1**) and (**2**) can be a small molecule, protein, polypeptide, peptide, antibody, polynucleotide, oligonucleotide, other polymeric species, polypeptide side chain or a biologically relevant targeting moiety, or a fragment, dimer, trimer or oligomer thereof.

In some embodiments, (**1**) and (**2**) are simultaneously mixed with a heterobifunctional PEG. In other embodiments, (**1**) and (**2**) are sequentially mixed with a heterobifunctional PEG.

In some embodiments, (**1**) is a cationic polymer. In some embodiments, (**2**) is a targeting moiety. In a preferred embodiment, (**1**) is a cationic polymer and (**2**) is a targeting moiety.

In another aspect, the present invention provides a method of using a heterobifunctional PEG to form a vehicle for targeted nucleic acid delivery of formula (XVII): 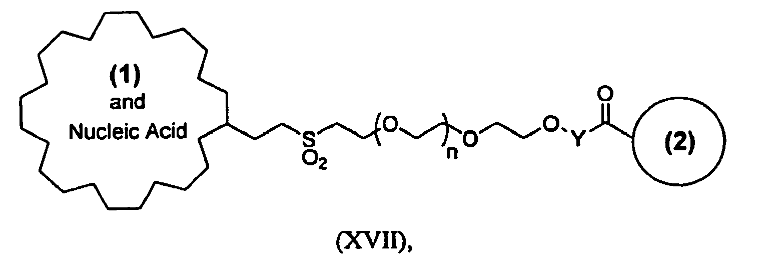 comprising the steps of (a) mixing a heterobifunctional PEG of the present invention with (**1**) and (**2**); and (b) mixing with a nucleic acid, wherein (**1**) is a cationic polymer and (2) is a targeting moiety.

In some embodiments, (**1**) and (**2**) are simultaneously mixed with heterobifunctional PEG. In other embodiments, (**1**) and (**2**) are sequentially mixed with heterobifunctional PEG.

### Detailed Description of the Invention

In order that the invention herein described may be fully understood, the following detailed description is set forth. The present invention provides heterobifunctional PEG reagents containing terminal vinyl sulfone and ester functionalities. These heterobifunctional PEGs are useful for a variety of reasons described above. Advantageously, the vinyl sulfone and ester functionalities provide different reactivities such that a reaction, e.g., with a cationic polymer, can occur selectively at the vinyl sulfone terminus.

In one aspect, the present invention provides a reagent comprising a compound of the formula (I): 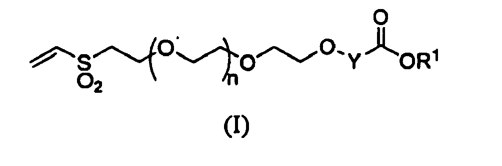 wherein:
Y is selected from the group consisting of C₁-C₆ alkylene, C₂-C₆ alkenylene, and C₂-C₆ alkynylene, wherein Y is optionally substituted with one or more substituents R²;
R² is independently selected from the group consisting of branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
R¹ is C₆-C₁₄ aryl, or C₅-C₁₄ heteroaryl containing one or more heteroatoms selected from the group consisting of N, N(R⁷), O, S, and S(O)ₘ, wherein m is 1 or 2, and wherein R¹ is optionally substituted with one or more substituents R⁸;
R⁷ is selected from the group consisting of H, branched or straight-chain C₁-C₆ alkyl, branched or straight-chain C₂-C₆ alkenyl, and branched or straight-chain C₂-C₆ alkynyl, wherein one or more hydrogens in the alkyl, alkenyl or alkynyl chain may be replaced by one or more fluorines;
R⁸ is independently selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl)₂, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines; and
n is an integer from 1 to 1,500.

In certain embodiments of compound (I), n is preferably an integer from 5 to 1,000. In certain embodiments, n is preferably from 20 to 500. In certain other embodiments, n is preferably from 50 to 250.

In other embodiments of compound (I), Y is preferably methylene. In certain embodiments, R¹ is preferably selected from the group consisting of phenyl, pyrimidinyl, pyridyl, and naphthyl; and R¹ is optionally substituted by one or more substituents R⁸. In certain embodiments, R¹ is preferably phenyl optionally substituted by one or more substituents R⁸. In certain embodiments, R¹ is phenyl and R⁸ is nitro.

The PEG reagents of compound (I) and their precursors can be synthesized from the appropriate sized unfunctionalized or partially functionalized polyethylene glycol (PEG) as illustrated below in Scheme I. The particular reactions may be carried out according to methods well-known in the literature.

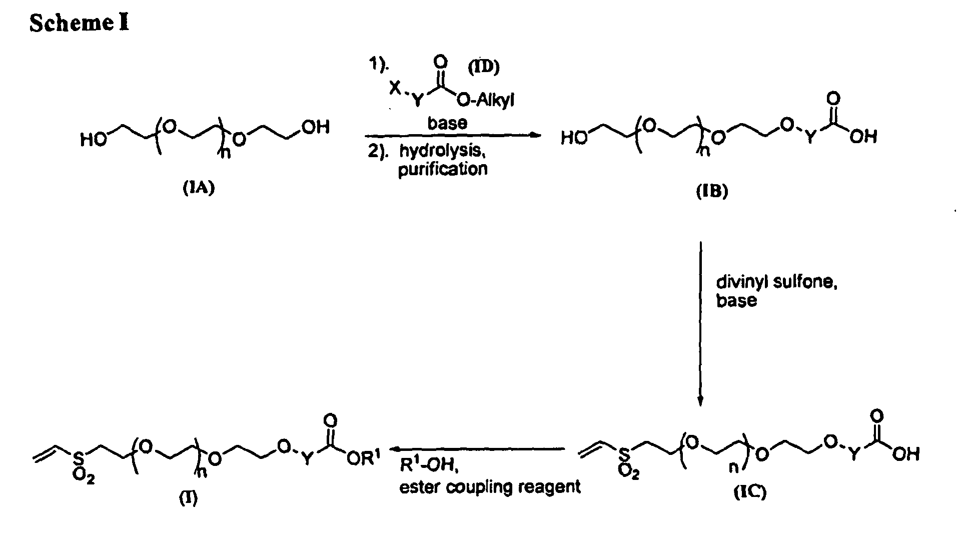

In Scheme I, compound (**IA**) is reacted with a base and compound (**ID**) to provide the corresponding alkyl ester PEG. The alkyl ester is subsequently hydrolyzed to provide compound (**IB**). While hydrolysis may produce a mixture of unfunctionalized, di-functionalized and mono-functionalized PEGs, the mono-functionalized PEG can be isolated via purification. (See, *e.g*., Zalipsky et al. J. Bioactive and Compatible Polymers, Vol. 5, 227-231, (1990)).

Compound (**IB**) is then reacted with divinyl sulfone in the presence of base to provide compound (**IC**). Next, compound (**IC**) is reacted with R¹-OH and a coupling reagent to provide compound (**I**).

Alternatively, compound (**I**) is prepared in two steps. First, R¹-OH is reacted with an activating agent and then reacted with compound (**IC**) to form compound (**I**). Suitable activating agents include, but are not limited to, methane sulfonyl chloride, tosyl chloride, trifluoroacetic anhydride and trifluoroacetyl chloride. In certain embodiments, the activating agent is methane sulfonyl chloride. In certain embodiments, the activating agent is tosyl chloride. In certain embodiments, the activating agent is trifluoroacetic anhydride. In certain embodiments, the activating agent is trifluoroacetyl chloride.

Alternatively, compound (**IB**) is reacted with an activating group and then a mercapto alkylalcohol. Suitable activating groups include, but are not limited to, methane sulfonyl chloride and tosyl chloride. The resulting thioether is oxidized and dehydrated to provide compound (**IC**).

In another aspect, the present invention provides a reagent comprising a compound of formula (II): 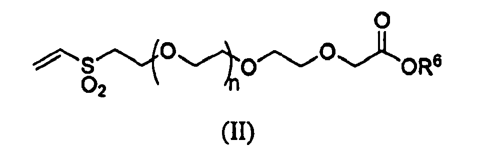 wherein R⁶ is H, branched or straight-chain C₁-C₆ alkyl, branched or straight-chain C₂-C₆ alkenyl, branched or straight-chain C₂-C₆ alkynyl; and
n is an integer from 1 to 1,500.

In certain embodiments, R⁶ is H. In other embodiments, R⁶ is alkyl. In some embodiments, R⁶ is methyl.

In certain embodiments, n is preferably an integer from 5 to 1,000. In certain embodiments, n is preferably from 20 to 500. In certain other embodiments, n is preferably from 50 to 250.

The PEG reagents of compound (II) and their precursors can be synthesized in a similar way as compound (I) as illustrated above in Scheme I.

In certain embodiments, compound (II) is a precursor to compound (IE) as illustrated below in Scheme II.

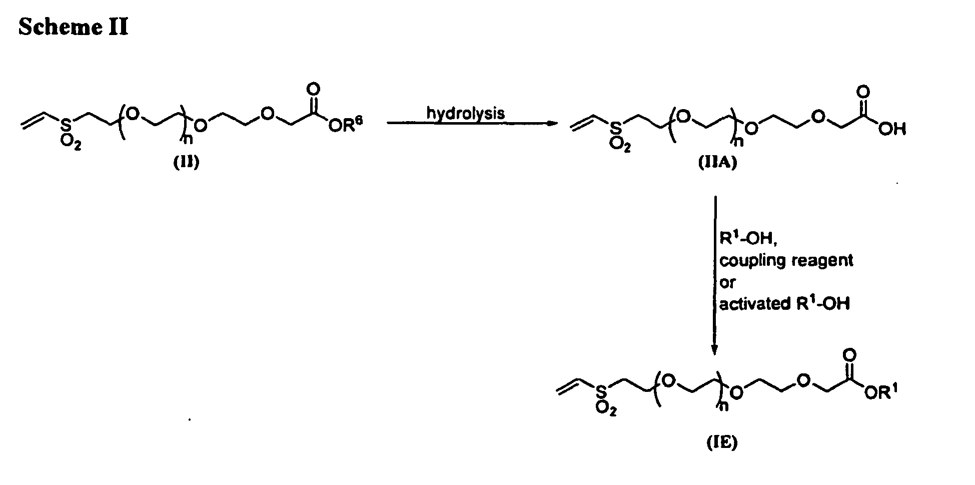

In Scheme **II**, compound (**II**) is hydrolyzed to produce compound (**IIA**). Compound (**IIA**) is subsequently reacted with R¹-OH and a coupling reagent to provide compound (**IE**).

Alternatively, compound (**IE**) is prepared in two steps. First, R'-OH is reacted with an activating agent and then reacted with compound (**IIA**) to form compound (IE). Suitable activating agents include, but are not limited to, methane sulfonyl chloride, tosyl chloride, trifluoroacetyl anhydride and trifluoroacetyl chloride. In certain embodiments, the activating agent is methane sulfonyl chloride. In certain embodiments, the activating agent is tosyl chloride. In certain embodiments, the activating agent is trifluoroacetyl anhydride. In certain embodiments, the activating agent is trifluoroacetyl chloride.

In another aspect, the present invention provides a method for producing heterobifunctional PEG reagents. This method is particularly useful because it efficiently provides heterobifunctional PEG reagents containing terminal vinyl sulfone and ester functionalities.

According to this aspect, the present invention provides a method for producing a heterobifunctional PEG compound of formula (XIV): 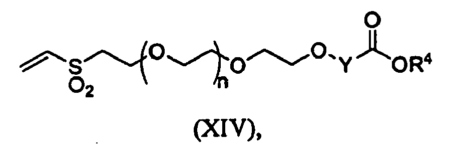 comprising the steps of:
(a) reacting a compound of formula (III) with divinyl sulfone: 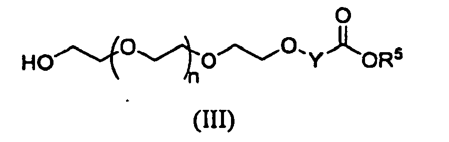 to form a compound of formula (XV): 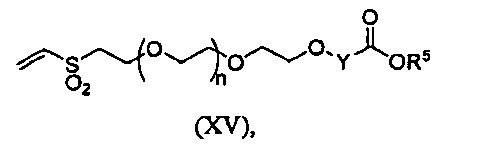 and (b) reacting the compound of formula (XV) with HO-R⁴ in the presence of an ester coupling agent to form the compound of formula (XIV),
   wherein:
   Y is selected from the group consisting of C₁-C₆ alkylene, C₂-C₆ alkenylene, and C₂-C₆ alkynylene, wherein Y is optionally substituted with one or more substituents R²;
   R² is independently selected from the group consisting of branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   n is an integer from 1 to 1,500,
   R⁵ is H;
   R⁴ is C₆-C₁₄ aryl or C₅-C₁₄ heteroaryl containing one or more heteroatoms selected from the group consisting of N, N(R⁷), O, S, and S(O)ₘ, wherein m is 1 or 2, wherein R⁴ is optionally substituted with one or more substituents R⁸;
   R⁷ is selected from the group consisting of H, branched or straight-chain C₁-C₆ alkyl, branched or straight-chain C₂-C₆ alkenyl, branched or straight-chain C₂-C₆ alkynyl, wherein one or more hydrogens in the alkyl, alkenyl or alkynyl chain may be replaced by one or more fluorines; and
   R⁸ is independently selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines.

In certain embodiments, n is preferably an integer from 5 to 1,000. In certain embodiments, n is preferably an integer from 20 to 500. In certain embodiments, n is preferably an integer from 50 to 250.

In certain embodiments, R⁴ is preferably phenyl, pyridyl, pyrimidinyl, or naphthyl; and R⁴ is optionally substituted by one or more substituents R⁸. In certain embodiments, R⁴ is preferably phenyl optionally substituted by one or more substituents R⁸. In certain embodiments of the method, R⁴ is phenyl and R⁸ is nitro.

In another aspect, the present invention provides a method for producing a compound of formula (VIII): 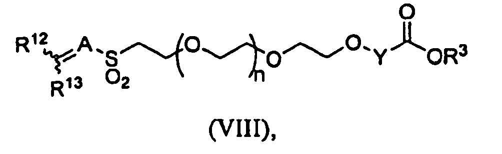 comprising the steps of:
(a) reacting a compound of formula (IV): 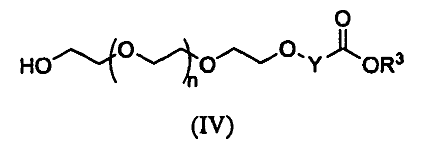 with an activating group to form a compound of formula (IVa): 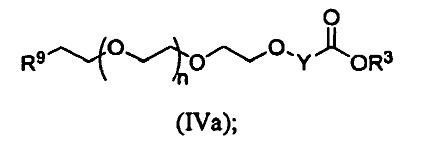
(b) reacting compound of formula (IVa) with a compound of formula (V): 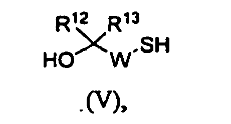 to form a compound of formula (VI): 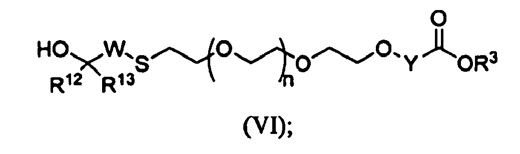
(c) oxidizing the compound of formula (VI) to form a compound of formula (VII): 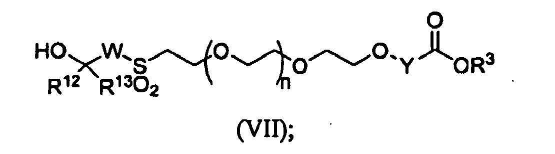 and (d) dehydrating the compound of formula (VII) to form a compound of formula (VIII): 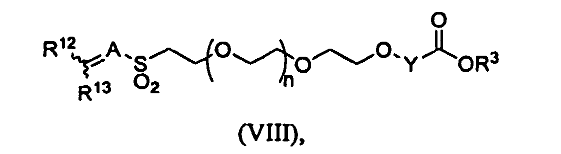 wherein:
   W is C₁-C₆ alkylene, wherein W is optionally substituted with one or more substituents R¹¹;
   A is C₁-C₆ 1-alkanyl-ylidene, wherein A is optionally substituted with one or more substituents R¹¹;
   R¹¹ is independently selected from the group consisting of H, branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   Y is selected from the group consisting of C₁-C₆ alkylene, C₂-C₆ alkenylene, and C₂-C₆ alkynylene, wherein Y is optionally substituted with one or more substituents R²; R² is independently selected from the group consisting of branched or straight-chain C₁-C₆ alkyl branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   R³ is H, R¹⁷, branched or straight-chain C₁-C₆ alkyl, branched or straight-chain C₂-C₆ alkenyl, branched or straight-chain C₂-C₆ alkynyl, C₆-C₁₄ aryl or C₅-C₁₄ heteroaryl containing one or more heteroatoms selected from the group consisting of N, N(R⁷), O, S, and S(O)ₘ, wherein m is 1 or 2, wherein one or more hydrogens in the alkyl, alkenyl or alkynyl chain may be replaced by one or more fluorines, provided that when R³ is not H or R¹⁷, R³ is optionally substituted with one or more substituents R⁸; R⁷ is selected from the group consisting of H, branched or straight-chain C₁-C₆ alkyl, branched or straight-chain C₂-C₆ alkenyl, and branched or straight-chain C₂-C₆ alkynyl, wherein one or more hydrogens in the alkyl, alkenyl or alkynyl chain may be replaced by one or more fluorines;
   R⁸ is selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl)₂, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   R¹⁷ is a protecting group;
   R⁹ is selected from the group consisting of CH₃S(O)₂-O-, CF₃S(O)₂-O-, CH₃(C₆H₄)S(O)₂-O-, Cl, Br, and I;
   R¹² is selected from the group consisting of H, branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   R¹³ is selected from the group consisting of H, branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines; and n is an integer from 1 to 1,500.

In certain embodiments, in compounds of formula (IV), R³ is H. In other embodiments in compounds of formula (IV), R³ is alkyl. In certain embodiments, R³ is methyl.

In certain embodiments, in the compounds of formula (IV), n is preferably an integer from 5 to 1,000. In certain embodiments, n is preferably an integer from 20 to 500. In certain embodiments, n is more preferably an integer from 50 to 250.

In certain embodiments in the compounds of formula (IV), R³ is selected from the group consisting of phenyl, pyridyl, pyrimidinyl, and naphthyl; and R³ is optionally substituted by one or more substituents R⁸. In certain more preferred embodiments, R³ is phenyl optionally substituted by one or more substituents R⁸. In certain more preferred embodiments, R³ is phenyl and R⁸ is nitro.

In certain embodiments, in the compound of formula (V), W is preferably methylene and A is methylidene.

Any suitable activating group may be used in step (a) to form a compound of formula (IVa). Suitable activating agents include, but are not limited to trifluoroacetic anhydride, 4-methylbenzoyl chloride, p-toluenesulfonyl chloride and methanesulfonyl chloride. In certain embodiments, the activating agent is trifluoroacetic anhydride. In certain embodiments, the activating agent is methanesulfonyl chloride.

Any suitable oxidizing agent may be used in step (c) to form a compound of formula (VII). Suitable oxidizing agents include, but are not limited to, hydrogen peroxide, m-chloroperbenzoic acid, potassium peroxomonosulfate, manganese dioxide, and potassium permanganate. The oxidizing agent may include other components and/or catalysts such as tungstic acid. In certain embodiments, the oxidizing agent is hydrogen peroxide in the presence of a tungstic acid catalyst.

Any suitable dehydration reaction may be used in step (d) to form a compound of formula (VIII). Suitable dehydration reactions include, but are not limited to, reaction with thionyl chloride and a base; reaction with methanesulfonyl chloride and a base; and reaction with Burgess' Reagent. In certain embodiments, the reaction is treatment with thionyl chloride and a base. In certain embodiments, the reaction is treatment with methanesulfonyl chloride and a base.

The method for producing a compound of formula (VIII) may further comprise the step of adding a protecting group to the carboxylic acid group on one or more compounds of formula IV, IVa, VI, VII, or VIII. Any suitable protecting group known in the art may be used. *See* e.g., Greene et al., Protecting Groups in Organic Synthesis (3rd ed.), John Wiley and Sons, New York (1999).

The method for producing a compound of formula (VIII) may further comprise the step of deprotecting the protecting group from one or more compounds of formula IV, IVa, VI, VII or VIII. The deprotecting step results in the corresponding compound of formula IV, IVa, VI, VII, or VII. The method may include more than one such deprotecting steps. Any suitable deprotection method known in the art may be used, e.g., hydrolysis.

In certain embodiments, the one or more protection steps can be carried out concurrently with any of steps (a)-(d). For example, a compound of formula IV, in which R³ is H, may be concurrently activated by the addition of R⁹, as well as protected at its free acid by the addition of a protecting group to produce a compound of formula IVa, in which R³ is R¹⁷. Analogously, any one or more deprotection steps can be carried out concurrently with any steps (a)-(d). For example, a compound of formula IV, in which R³ is not H, may be concurrently activated by the addition of R⁹, as well as deprotected with a suitable deprotection agent to produce the free acid of the compound of formula IVa, in which R³ is H.

In another aspect, the present invention provides a method for producing a reagent of formula (X): 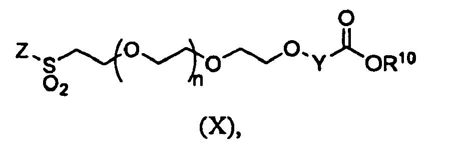 comprising the step of:
(a) reacting a compound of formula (IX): 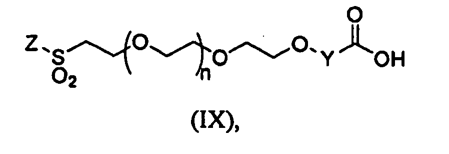 with an activating agent R¹⁰-R¹⁴ to form a compound of formula (X),
   wherein:
   R¹⁰ is a branched or straight-chain C₁-C₆ alkyl, branched or straight-chain C₂-C₆ alkenyl, branched or straight-chain C₂-C₆ alkynyl, C₆-C₁₄ aryl or C₅-C₁₄ heteroaryl containing one or more heteroatoms selected from the group consisting of N, N(R⁷);
   O, S, and S(O)ₘ, wherein m is 1 or 2, wherein R¹⁰ is optionally substituted with one or
   more substituents R⁸;
   R⁷ is selected from the group consisting of H, branched or straight-chain C₁-C₆ alkyl,
   branched or straight-chain C₂-C₆ alkenyl, and branched or straight-chain C₂-C₆ alkynyl, wherein one or more hydrogens in the alkyl, alkenyl or alkynyl chain may be replaced by one or more fluorines;
   R⁸ is independently selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl)₂, C₁-C₆ alkoxy, CO₂H,
   CO₂(C₁-C₆ alkyl), CONH₂, CONH(C₁-C₆ alkyl), CON(C₁-C₆ alkyl)₂, nitro, cyano,
   and halo, wherein one or more hydrogens in the alkyl, alkenyl or alkynyl chain may be replaced by one or more fluorines;
   R¹⁴ is selected from the group consisting of HO-, CF₃C(O)-O-, CH₃S(O)₂-O-, CF₃S(O)₂-O-, CH₃(C₆H₄)S(O)₂-O-, Cl, Br, and I;
   Z is selected from the group consisting of -CH₂CH₂Cl, -CH₂CH₂Br, -CH₂CH₂I, -CH₂CH₂OS(O)₂CH₃, -CH₂CH₂OS(O)₂CF₃, -CH₂CH₂OS(O)₂ (C₆H₄)CH₃, and -CH=CH₂;
   Y is selected from the group consisting of C₁-C₆ alkylene, C₂-C₆ alkenylene, and C₂-C₆ alkynylene, wherein Y is optionally substituted with one or more substituents R²,
   R² is independently selected from the group consisting of branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines; and n is an integer from 1 to 1,500.

In certain embodiments, Z is -CH=CH₂.

In certain embodiments, R¹⁰ is preferably phenyl, R⁸ is nitro and R¹⁴ is CF₃CO-O-.

In certain preferred embodiments, R¹⁰ is phenyl, R⁸ is nitro and R¹⁴ is HO-and the activating reagent further comprises an ester-coupling reagent.

In certain embodiments, n is preferably an integer from 5 to 1,000. In certain embodiments, n is preferably an integer from 20 to 500. In certain embodiments, n is more preferably an integer from 50 to 250.

In certain other embodiments, R¹⁰ is selected from the group consisting of phenyl, pyridyl, pyrimidinyl, and naphthyl; and R¹⁰ is optionally substituted by one or more substituents R⁸. In certain preferred embodiments, R¹⁰ is phenyl optionally substituted by one or more substituents R⁸. In certain more preferred embodiments, R¹⁰ is phenyl and R⁸ is nitro.

When R¹⁴ is HO-, any suitable coupling agent may be used in step (a). Suitable coupling reagents include, but are not limited to, DCC (1,3-Dicyclohexylcarbodiimide), EDC (1-(3-Dimethylaminopropyl)-3-ethylcarbodiimide hydrochloride), HATU (O-(7-Azabenzotriazol-1-yl)-N,N,N',N'-tetramethyluronium hexafluorophosphate), and HBTU (O-Benzotriazol-1-yl-N,N,N',N'-tetramethyluronium hexafluorophosphate). In certain embodiments, the ester-coupling reagent is DCC.

In another aspect, the present invention further provides a method for producing a compound of formula (XIII): 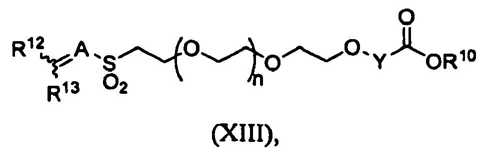 comprising the steps of:
(a) reacting a compound of formula (XI) 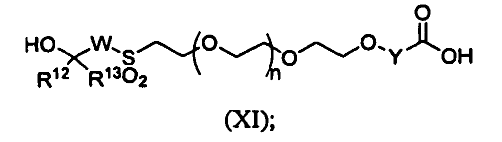 with an activating reagent to form a compound of formula (XII), wherein the activating reagent comprises R¹⁰-R¹⁴; 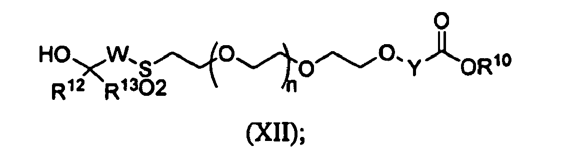 and (b) dehydrating the compound of formula (XII) to form a compound of formula (XIII),
   wherein:
   W is C₁-C₆ alkylene, wherein W is optionally substituted with one or more substituents R¹¹;
   A is C₁-C₆ 1-alkanyl-ylidene, wherein A is optionally substituted with one or more substituents R¹¹;
   R¹¹ is independently selected from the group consisting of H, branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   Y is selected from the group consisting of C₁-C₆ alkylene, C₂-C₆ alkenylene, and C₂-C₆ alkynylene, wherein Y is optionally substituted with one or more substituents R²; R² is independently selected from the group consisting of branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   R¹⁰ is a branched or straight-chain C₁-C₆ alkyl, branched or straight-chain C₂-C₆ alkenyl, branched or straight-chain C₂-C₆ alkynyl, C₆-C₁₄ aryl or C₅-C₁₄ heteroaryl containing one or more heteroatoms selected from the group consisting ofN, N(R⁷), O, S, and S(O)ₘ, wherein m is 1 or 2, wherein R¹⁰ is optionally substituted with one or more substituents R⁸;
   R⁷ is selected from the group consisting of H, branched or straight-chain C₁-C₆ alkyl, branched or straight-chain C₂-C₆ alkenyl, and branched or straight-chain C₂-C₆ alkynyl, wherein one or more hydrogens in the alkyl, alkenyl or alkynyl chain may be replaced by one or more fluorines;
   R⁸ is independently selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl)₂, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   R¹⁴ is selected from the group consisting of HO-, CF₃C(O)-O-, CH₃S(O)₂-O-, CF₃S(O)₂-O-, CH₃(C₆H₄)S(O)₂-O-, Cl, Br, and I;
   R¹² is selected from the group consisting of H, branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
   R¹³ is selected from the group consisting of H, branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl, nitro, cyano, and halo, wherein one or more hydrogens in the alkyl, alkenyl or alkynyl chain may be replaced by one or more fluorines; and n is an integer from 1 to 1,500.

In certain embodiments, R¹⁰ is phenyl, R⁸ is nitro and R¹⁴ is CF₃-CO-O-.

In certain embodiments, R¹⁰ is phenyl, R⁸ is nitro, R¹⁴ is HO- and the activating reagent further comprises an ester-coupling reagent.

In certain other embodiments, R¹⁰ is selected from the group consisting of phenyl, pyridyl, pyrimidinyl, and naphthyl; and R¹⁰ is optionally substituted by one or more substituents R⁸. In certain preferred embodiments, R¹⁰ is phenyl optionally substituted by one or more substituents R⁸. In certain more preferred embodiments, R¹⁰ is phenyl and R⁸ is nitro.

In certain embodiments, W is methylene. In certain embodiments, A is methylidene.

When R¹⁴ is HO-, any suitable ester-coupling agent may be used in step (a). Suitable ester-coupling reagents include, but are not limited to, DCC (1,3-Dicyclohexylcarbodiimide), EDC (1-(3-Dimethylaminopropyl)-3-ethylcarbodiimide hydrochloride), HATU (O-(7-Azabenzotriazol-1-yl)-N,N,N',N'-tetramethyluronium hexafluorophosphate), and HBTU (O-Benzotriazol-1-yl-N,N,N',N'-tetramethyluronium hexafluorophosphate). In certain more preferred embodiments, the ester-coupling reagent is DCC.

Any suitable dehydration reaction may be used in step (b). Suitable dehydration reactions include, but are not limited to, reaction with thionyl chloride and a base; reaction with methanesulfonyl chloride and a base; and reaction with Burgess' Reagent. In certain embodiments, the reaction is treatment with thionyl chloride and a base. In certain embodiments, the reaction is treatment with methanesulfonyl chloride and a base.

In another aspect, the present invention provides a method of using a heterobifunctional PEG to form a vector of formula (XVI): 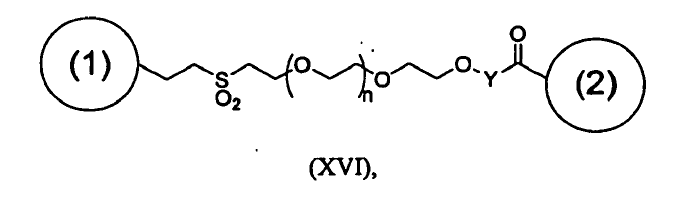 comprising the step of mixing a heterobifunctional PEG of the present invention with (**1**) and (**2**), wherein (**1**) and (**2**) can be a small molecule, protein, polypeptide, peptide, antibody, polynucleotide, oligonucleotide, other polymeric species, polypeptide side chain or a biologically relevant targeting moiety, or a fragment, dimer, trimer or oligomer thereof.

In some embodiments, (**1**) and (**2**) are simultaneously mixed with a heterobifunctional PEG. In other embodiments, (**1**) and (**2**) are sequentially mixed with a heterobifunctional PEG.

In some embodiments, (**1**) is a cationic polymer. In some embodiments, (**2**) is a targeting moiety. In a preferred embodiment, (**1**) is a cationic polymer and (**2**) is a targeting moiety.

In another aspect, the present invention provides a method of using a heterobifunctional PEG to form a vehicle for targeted nucleic acid delivery of formula (XVII): 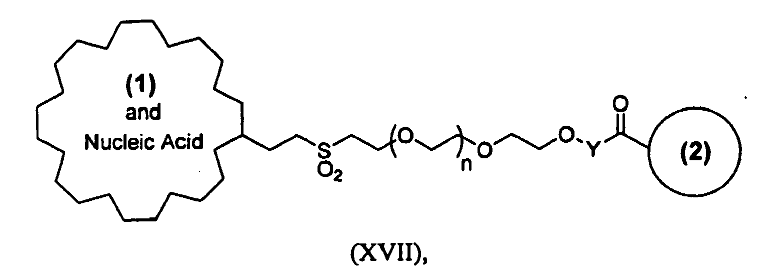 comprising the steps of (a) mixing a heterobifunctional PEG of the present invention with (**1**) and (**2**); and (b) mixing with a nucleic acid, wherein (**1**) is a cationic polymer and (**2**) is a targeting moiety.

In some embodiments, (**1**) and (**2**) are simultaneously mixed with a heterobifunctional PEG. In other embodiments, (**1**) and (**2**) are sequentially mixed with a heterobifunctional PEG.

In some embodiments, the heterobifunctional PEG regent is a compound of formula (I). In other embodiments, the heterobifunctional PEG reagent is compound of formula (XIV). In other embodiments, the heterobifunctional PEG reagent is compound of formula (VIII).

Suitable nucleic acids include, but are not limited to, a recombinant plasmid; a replication-deficient plasmid; a mini-plasmid lacking bacterial sequences; a recombinant viral genome; a linear nucleic acid fragment encoding a therapeutic peptide or protein; a hybrid DNA/RNA double strand; double stranded DNA; an antisense DNA or chemical analogue thereof; a blunt, double blunt and overhanging double stranded DNA or RNA fragment comprising 5-200 base pairs; an antisense RNA or chemical analogue thereof; a linear polynucleotide that is transcribed as an antisense RNA or a ribozyme; a ribozyme; and a viral genome.

In certain embodiments, the blunt, double blunt and overhanging double stranded DNA or RNA comprises 15-30 base pairs. In certain embodiments, the blunt, double blunt and overhanging double stranded DNA or RNA comprises 15 base pairs. In certain embodiments, the blunt, double blunt and overhanging double stranded DNA or RNA comprises 16 base pairs. In certain embodiments, the blunt, double blunt and overhanging double stranded DNA or RNA comprises 17 base pairs. In certain embodiments, the blunt, double blunt and overhanging double stranded DNA or RNA comprises 18 base pairs. In certain embodiments, the blunt, double blunt and overhanging double stranded DNA or RNA comprises 19 base pairs. In certain embodiments, the blunt, double blunt and overhanging double stranded DNA branches. In certain even more preferred embodiments, the branched HK copolymer contains 4 branches.

In some embodiments, the branch of the branched HK copolymer comprises 10-100 amino acid residues. In certain preferred embodiments, the branch comprises 10-50 amino acid residues. In certain more preferred embodiments, the branch comprises 15-25 amino acid residues. In certain embodiments, the branch of the branched HK copolymer comprises at least 3 histidine amino acid residues in every subsegment of 5 amino acid residues. In certain other embodiments, the branch comprises at least 3 histidine amino acid residues in every subsegment of 4 amino acid residues. In certain other embodiments, the branch comprises at least 2 histidine amino acid residues in every subsegment of 3 amino acid residues. In certain other embodiments, the branch comprises at least 1 histidine amino acid residues in every subsegment of 2 amino acid residues.

In certain embodiments, at least 50% of the branch of the HK copolymer comprises units of the sequence KHHH. In certain preferred embodiments, at least 75% of the branch comprises units of the sequence KHHH.

In certain embodiments, the HK copolymer branch comprises an amino acid residue other than histidine or lysine. In certain preferred embodiments, the branch comprises a cysteine amino acid residue, wherein the cysteine is a N-terminal amino acid residue.

In certain embodiments, suitable HK copolymers include, but are not limited to those found in U.S. Patent Nos. 6,692,911, 7,070,807 and 7,163,695, all of which are incorporated herein by reference.

Any biologically relevant targeting moiety may be used in the vectors of the present invention. In certain embodiments, the targeting moiety is a small molecule, polypeptide, peptide, protein, antibody, or a fragment, dimer, trimer or oligomer thereof. Suitable biologically relevant small molecule targeting moieties include, but are not limited to, vascular endothelial cell growth factor for targeting endothelial cells; FGF2 for targeting vascular lesions and tumors; transferrin for targeting tumors; melanotropin (alpha MSH) peptides for tumor targeting; ApoE and peptides for LDL receptor targeting; von Willebrand's Factor and peptides for targeting Coxsackie-adenoviral receptor (CAR) expressing cells; PD1 and peptides for targeting Neuropilin 1; EGF and peptides for targeting EGF receptors expressing cells; folic acid and ligands for targeting folate receptors; RGD peptides for targeting integrin or RNA comprises 20 base pairs. In certain embodiments, the blunt, double blunt and overhanging double stranded DNA or RNA comprises 21 base pairs. In certain embodiments, the blunt, double blunt and overhanging double stranded DNA or RNA comprises 22 base pairs. In certain embodiments, the blunt, double blunt and overhanging double stranded DNA or RNA comprises 23 base pairs. In certain embodiments, the blunt, double blunt and overhanging double stranded DNA or RNA comprises 24 base pairs. In certain embodiments, the blunt, double blunt and overhanging double stranded DNA or RNA comprises 25 base pairs. In certain embodiments, the blunt, double blunt and overhanging double stranded DNA or RNA comprises 26 base pairs. In certain embodiments, the blunt, double blunt and overhanging double stranded DNA or RNA comprises 27 base pairs. In certain embodiments, the blunt, double blunt and overhanging double stranded DNA or RNA comprises 28 base pairs. In certain embodiments, the blunt, double blunt and overhanging double stranded DNA or RNA comprises 29 base pairs. In certain embodiments, the blunt, double blunt and overhanging double stranded DNA or RNA comprises 30 base pairs.

Suitable cationic polymers useful for forming a vector include, but are not limited to, linear or branched HK copolymers (copolymers of histidine and lysine), linear or branched polyethyleneimine (PEI), polylysine, linear or non-linear polyamidoamine, protamine sulfate, polybrine, chitosan, polymethacrylate, polyamines, spermine analogues and any other suitable polymer.

A preferred cationic polymer is an HK copolymer. In certain embodiments, the HK copolymer is synthesized from any appropriate combination of polyhistidine, polylysine, histidine and/or lysine. In certain embodiments, the HK copolymer is linear. In certain preferred embodiments, the HK copolymer is branched.

In certain preferred embodiments, the branched HK copolymer comprises a polypeptide backbone. Preferably, the polypeptide backbone comprises 1-10 amino acid residues, and more preferably 2-5 amino acid residues.

In certain preferred embodiments, the polypeptide backbone consists of lysine amino acid residues.

In certain preferred embodiments, the number of branches on the branched HK copolymer is one greater than the number of backbone amino acid residues. In certain preferred embodiments, the branched HK copolymer contains 1-11 branches. In certain more preferred embodiments, the branched HK copolymer contains 2-5 expressing cells and any other suitable targeting moiety. Additionally, any fragments or peptides of the above moieties having the same or similar targeting properties may be used as for their respective targets.

Unless defined otherwise, all technical and scientific terms used herein have the same meaning as those commonly understood by one or ordinary in the art to which this invention belongs. Although methods and materials similar or equivalent to those described herein can be used in the practice or testing of the present invention, suitable methods and materials are described herein. These materials, methods and examples are illustrative only, and are not intended to be limiting. All publications, patents and other documents mentioned herein are incorporated by reference in their entirety.

Throughout this specification, the word "comprise" or variations such as "comprises" or "comprising" will be understood to imply the inclusion of a stated integer or group of integers but not the exclusion of any other integer or group of integers.

In order to further define the invention, the following terms and definitions are provided. As used above, and throughout the specification, the following terms, unless otherwise indicated, shall be understood to have the following meanings:

"Alkyl" means an aliphatic hydrocarbon group which may be a straight or branched chain and comprising about 1 to 6 carbon atoms in the chain. Branched means that one or more alkyl groups such as methyl, ethyl, or propyl, are attached to a linear alkyl chain.

"Alkenyl" means an aliphatic hydrocarbon group which may be a straight or branched chain and containing at least one carbon-carbon double bond. Branched means that one or more lower alkyl groups such as methyl, ethyl or propyl are attached to a linear alkenyl chain.

"Alkynyl" means an aliphatic hydrocarbon group and which may be a straight or branched chain and containing at least one carbon-carbon triple bond. Branched means that one or more lower alkyl groups such as methyl, ethyl or propyl are attached to a linear alkynyl chain.

"Alkylene" means a divalent alkyl group obtained by removal of a hydrogen atom from an alkyl group that is defined above.

"Aryl" means an aromatic monocyclic or multicyclic ring system containing conjugated double bonds, wherein the π-electrons associated with the double bonds, in conjunction with any participating lone pairs, positive or negative charges, fulfills the 2n+2 formula, wherein n is a whole number. Examples of aryl groups include, but are not limited to, phenyl and naphthalene.

"Heteroaryl" means an aromatic monocyclic or multicyclic ring system in which one or more of the ring atoms is an element other than carbon, for example nitrogen, oxygen, or sulfur, alone or in combination. The prefix aza, oxa or thia before the heteroaryl root name means that at least nitrogen, oxygen, or sulfur atom respectively, is present as a ring atom. Suitable heteraryl groups include, but are not limited to, pyridine, pyrole, furan, and thiophene.

"Alkenylene" means a linear or branched divalent hydrocarbon radical of two to ten carbons containing at least one unsaturated double bond in the hydrocarbon chain. Branched means that one or more lower alkyl groups such as methyl, ethyl or propyl are attached to a linear alkenylene chain.

"Alkynylene" means a linear or branched divalent hydrocarbon radical of two to ten carbons containing at least one unsaturated triple bond in the hydrocarbon chain. Branched means that one or more lower alkyl groups such as methyl, ethyl or propyl are attached to a linear alkynylene chain.

"Halo" means a fluorine, chlorine, bromine or iodine substituent.

"Protecting group" means a group used in organic synthesis to temporarily mask the characteristic chemistry of a select functional group. Suitable protecting groups for the methods and compounds described herein include, but are not limited to, those described in standard textbooks, such as Greene, T. W. et al., Protective Groups in Organic Synthesis, Wiley, N.Y. (1999).

An "activating group" means a moiety attached to a hydroxy group that allows for a more facial nucleophilic substitution on the adjacent carbon atom. Suitable examples include, but are not limited to, a methane sulfonyl group and a trifluoroacetate group.

While we have hereinbefore presented a number of embodiments of this invention, it is apparent that our basic construction can be altered to provide other embodiments which utilize the methods of this invention. Therefore, it will be appreciated that the scope of this invention is to be defined by the claims appended hereto rather than the specific embodiments which have been presented herein by way of example.

### Examples

### Example 1: Preparation of PEG-3400-Methyl Ester.

Acetyl chloride (2.50 mL, 35.16 mmoles) was added to 250 mL of methanol. This mixture was stirred for 5 min and then added to PEG-3400-Monocarboxylic acid (20.01 g, 5.81 mmoles) as prepared in Zalipsky et al. (J. Bioactive and Compatible Polymers, Vol. 5, 227-231, (1990)). After stirring at room temperature for 24 hours, the resulting solution was concentrated to dryness. The residue was dissolved in methylene chloride (15 mL) and the resulting solution was added to ether (300 mL) with vigorous stirring. The resulting solids are filtered, washed with ether and dried under vacuum yielding the desired PEG-3400 methyl ester (18.93 g, 94% yield) as a white powder.

### Example 2: Preparation of PEG-3400 Methyl Ester Mesylate.

The PEG-3400 Methyl Ester product (18.93 g, 5.45 mmoles) of Example 1 was dissolved in methylene chloride (100 mL). Diisopropylethylamine (3.80 mL, 21.81 mmoles) was added followed by methanesulfonyl chloride (1.27 mL, 16.36 mmoles). The reaction was stirred at room temperature for 18 hours after which, it was partitioned with a mixture of brine (90 mL), water (90 mL) and 6 M HCl (20 mL). The layers were separated and the aqueous layer was washed with methylene chloride (3 X 60 mL). The combined organic layers were dried over anhydrous magnesium sulfate, filtered and concentrated to approximately 20 mL. The resulting material was poured into ether (300 mL) with rapid stirring. The resulting solids were filtered, washed with ether and dried under vacuum providing the desired product (18.99 g, 98%) as an off white to light brown powder.

### Example 3: Preparation of PEG-3400 Monocarboxylic acid hydroxyethylsulfide.

The PEG-3400 Methyl Ester mesylate (18.99 g, 5.19 mmoles) product from Example 2 was added to a mixture of water (150 mL) and mercaptoethanol (2.92 mL, 41.55 mmoles). 2 M sodium hydroxide (20.80 mL, 41.60 mmoles) was subsequently added to the mixture. The mixture was heated to reflux and stirred for 3 hours after which it was cooled to room temperature. Sodium chloride (50 g) was added and the mixture was acidified using 6 M HCl. The mixture was then washed with methylene chloride (3 X 60 mL). The combined organic extracts were dried over anhydrous magnesium sulfate, filtered and concentrated to approximately 20 mL. The residue was poured into ether (300 mL) with rapid stirring. The resulting solids were filtered, washed with ether and dried under vacuum providing the desired product (17.76 g, 97% yield) as a white powder.

### Example 4: Preparation of PEG-3400 Monocarboxylic acid hydroxyethylsulfone.

The PEG-3400 Monocarboxylic acid mercaptoethanol product (17.76 g, 5.04 mmoles) from Example 3 was dissolved in water (40 mL). Tungstic acid (1.19 g, 4.76 mmoles) was added followed by water (30 mL). A 30% solution of hydrogen peroxide in water (2.06 mL) was added and the reaction was stirred at room temperature for 20 hours. The solids were filtered from the reaction and sodium chloride (20 g) was dissolved in the filtrate. The resulting solution was washed with methylene chloride (3 X 60 mL). The combined organics were dried over anhydrous magnesium sulfate, filtered and concentrated to approximately 20 mL. The residue was added to ether (300 mL) with rapid stirring. The resulting solids were filtered, washed with ether and dried under vacuum giving the desired product (17.65 g, 98% yield).

### Example 5: Preparation of PEG-3400 Methyl ester hydroxyethylsulfone.

Acetyl chloride (2.50 mL, 35.16 mmoles) was added to methanol (250 mL) and stirred at room temperature for 5 minutes. The PEG-3400 monocarboxylic acid hydroxyethylsulfone product from Example 4 (17.65 g, 4.97 mmoles) was then added to the mixture. After stirring at room temperature for 3 days, the resulting solution was concentrated to dryness. The residue was dissolved in methylene chloride (20 mL) and the resulting solution was added to ether (300 mL) with rapid stirring. The resulting solids were filtered, washed with ether and dried under vacuum yielding the desired methyl ester (17.08 g, 96% yield) as a white powder.

**Example 6: Preparation of PEG-3400 Methyl ester vinyl sulfone.** The PEG-3400 methyl ester hydroxyethylsulfone product (17.08 g, 4.79 mmoles) from Example 5 was dissolved in methylene chloride (115 mL). 2,6-Di-*tert*-butyl-4-methylphenol (10.6 mg, 0.048 mmole) and diisopropylethylamine (5.00 mL, 28.75 mmoles) were added followed by methanesulfonyl chloride (1.12 mL, 14.38 mmoles). The reaction was stirred at room temperature for 17 hours after which, it was partitioned with a mixture of brine (135 mL), water (135 mL) and 6 M HCl (30 mL). The layers were separated and the aqueous layer was washed with methylene chloride (3 X 60 mL). The combined organic extracts were dried over anhydrous magnesium sulfate, filtered and concentrated to approximately 20 mL. The residue was poured into ether (300 mL) with rapid stirring. The solids were filtered, washed with ether and dried under vacuum giving the desired product (16.64 g, 98% yield) as an off white to light brown powder.

**Example 7: Preparation of PEG-3400 Monocarboxylic acid vinyl** sulfone. NaOH (1 M in H₂O, 7.04 mL) was added to H₂O (340 mL) and the diluted NaOH solution was added to the methyl ester vinyl sulfone product (16.64 g, 4.69 mmoles) from Example 6. The reaction was stirred at room temperature for 15 minutes after which, it was acidified with 6 M HCl. Sodium chloride (96 g) was dissolved into the mixture and the product was extracted with methylene chloride (5 X 60 mL). The combined organic extracts were dried over anhydrous magnesium sulfate, filtered and concentrated to approximately 20 mL. The residue was poured into ether (300 mL) with rapid stirring. The solids were filtered, washed with ether and dried under vacuum giving the desired product (16.00 g, 97% yield) as an off white to light brown powder.

### Example 8: Preparation of PEG-3400 p-Nitrophenyl ester vinyl sulfone (DCC Method).

Dicyclohexylcarbodiimide (1.40 mg, 6.80 mmoles) was dissolved in methylene chloride (55 mL) and 4-nitrophenol (2.85 g, 20.39 mmoles) was added. The mixture was stirred at room temperature for 30 minutes after which, the PEG-3400 monocarboxylic acid vinyl sulfone product (16.00 g, 4.53 mmole) from Example 7 and 2,6-Di-*tert-*butyl-4-methylphenol (10.0 mg, 0.045 mmoles) were added. The reaction was stirred at room temperature for 22 hours after which, it was diluted with ethyl acetate (97 mL). The resulting precipitate was filtered and the filtrate was washed with ethyl acetate (50 mL). The resulting solution was concentrated to approximately 50 mL. The resulting precipitate was filtered and washed with ethyl acetate (20 mL). The resulting solution was poured into ether (300 mL) with rapid stirring. The resulting solids were collected by filtration and washed with ether, and dried under vacuum. The solids were recrystallized from isopropyl alcohol (100 mL) containing 2,6-Di-*tert-*butyl-4-methylphenol (12.4 mg) giving the desired product (15.87 g, 96% yield) as an off white to light brown solid.

### Example 9: Preparation of PEG-3400 p-Nitrophenyl ester vinyl sulfone (trifluoroacetyl nitrophenol method).

PEG-3400 monocarboxylic acid vinyl sulfone product (528.2 mg, 0.15 mmoles) prepared in accordance with Example 7 was dried under vacuum for 24 hours in a vacuum dessicator containing phosphorus pentoxide as a dessicant. The dried PEG-3400 monocarboxylic acid vinyl sulfone (5.35 g, 1.51 mmoles) and 2,6-Di-tert-butyl-4-methylphenol (3.34 mg, 0.015 mmoles) were dissolved in anhydrous pyridine (5.35 mL). Trifluoroacetyl nitrophenol (1.60 g, 6.81 mmoles) was added and the reaction was stirred at room temperature for 30 minutes. The mixture was then poured into ether (300 mL) with rapid stirring. The resulting solids were filtered, washed with ether and dried under vacuum giving the desired product (5.42 g, 98% yield) as an off white to light brown solid.

### Example 10: Preparation of PEG-8000 p-Nitrophenyl ester vinyl sulfone.

PEG-8000 Monocarboxylic acid was prepared essentially as described in Zalipsky et al. (J. Bioactive and Compatible Polymers, 5:227-231 (1990)). PEG-8000 p-nitrophenyl ester vinyl sulfone was prepared according to previously described Examples 1-7 and 9.

**Example 11: Preparation of Mono-carboxylic acid vinyl sulfone.** PEG-3400-Monocarboxylic acid (20.0 g, 5.80 mmoles) as prepared in Zalipsky et al. (J. Bioactive and Compatible Polymers, Vol. 5, 227-231, (1990)) was azeotropically dried in toluene (300 mL). During the drying process, toluene (240 mL) was removed by distillation and the resulting solution was cooled to room temperature. Anhydrous methylene chloride (600 mL) was added followed by sodium hydride (60%, 1.88 g, 47 mmoles). The reaction was stirred under helium at room temperature for 3 hours, after which divinyl sulfone (29.5 mL, 294 mmoles) was added. The reaction was stirred under helium at room temperature for 24-72 hours after which glacial acetic acid (5 mL) was added. Insoluble material was then removed by filtration and the filtrate was concentrated to a small volume. The residue was poured into cold ether. The resulting solids were filtered, dried under vacuum and dissolved in water (1000 mL) containing sodium chloride (5 g). The resulting solution was washed with methylene chloride (3 X 300 mL). The combined organic extracts were dried over anhydrous sodium sulfate, filtered and concentrated. The residue was poured into cold ether and the resulting solids were filtered and washed with cold ether. The resulting solids were dissolved in warm isopropanol (50 - 60 deg C) and the resulting solution was allowed to cool to room temperature overnight. The resulting solids were collected by filtration, washed with cold isopropanol and dried under vacuum providing the crude product (10-14 g, 50-70% yield).

A column (1 inch diameter) of DEAE-Sephadex (5 g) was treated with potassium tetraborate (10% solution in water) followed by water until the eluent attained a neutral pH. The crude product (4 g) was dissolved in water (35 mL) and applied to the column with the assistance of low nitrogen pressure. The column was eluted with water until the eluent contained no PEG-related material as determined using the polyacrylic acid (PAA) test. The column was then eluted with sodium chloride solution (8 mM in water) and fractions were collected until no PEG-related material was detected using the PAA test. Fractions containing the desired product were acidified with glacial acetic acid to pH 4-5 and the product was extracted into methylene chloride. The methylene chloride was dried over anhydrous sodium sulfate, filtered and concentrated. The residue was poured into cold ether and the resulting solids were filtered and dried under vacuum giving the desired product (2.2 g, 55% yield from column).

## Claims

1. A reagent comprising a compound of formula (I): 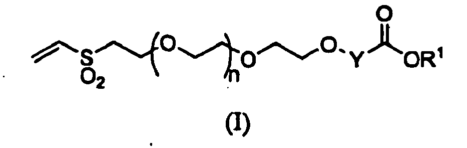 wherein:
Y is selected from the group consisting of C₁-C₆ alkylene, C₂-C₆ alkenylene, and C₂-C₆ alkynylene, wherein Y is optionally substituted with one or more substituents R²;
R² is independently selected from the group consisting of branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
R¹ is C₆-C₁₄ aryl, or C₅-C₁₄ heteroaryl containing one or more heteroatoms selected from the group consisting of N, N(R⁷), O, S, and S(O)ₘ, wherein m is 1 or 2, and wherein R¹ is optionally substituted with one or more substituents R⁸;
R⁷ is selected from the group consisting of H, branched or straight-chain C₁-C₆ alkyl, branched or straight-chain C₂-C₆ alkenyl, and branched or straight-chain C₂-C₆ alkynyl, wherein one or more hydrogens in the alkyl, alkenyl or alkynyl chain may be replaced by one or more fluorines;
R⁸ is independently selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl)₂, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines; and
n is an integer from 1 to 1,500.

2. The reagent of claim 1, wherein n is an integer from 5 to 1,000.

3. The reagent of claim 2, wherein n is an integer from 20 to 500.

4. The reagent of claim 3, wherein n is an integer from 50 to 250.

5. The reagent of claim 1, wherein Y is methylene.

6. The reagent of claim 1, wherein R¹ is selected from the group consisting of phenyl, pyridyl, pyrimidinyl, and naphthyl.

7. The reagent of claim 1, wherein R¹ is phenyl.

8. The reagent of claim 1, wherein R¹ is phenyl and R⁸ is nitro.

9. A method for producing a compound of formula (VIII), 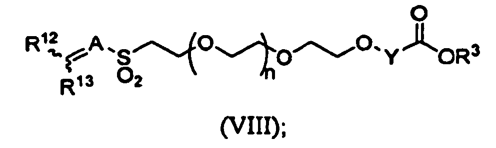 comprising the steps of:
(a) reacting a compound of formula (IV): 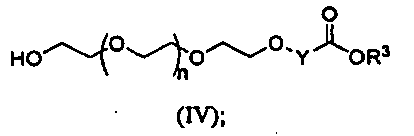 with an activating group to form a compound of formula (IVa): 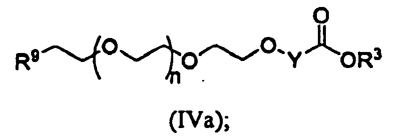
(b) reacting the compound of formula (IVa) with a compound of formula (V): 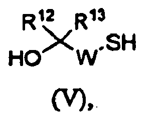 to form a compound of formula (VI): 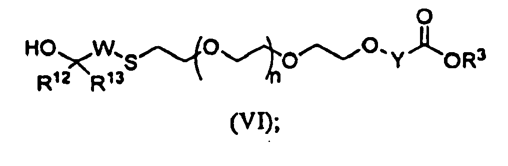
(c) oxidizing the compound of formula (VI) to form a compound of formula (VII): 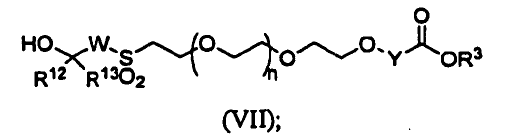
and (d) dehydrating the compound of formula (VII) to form a compound of formula (VIII),
wherein:
W is C₁-C₆ alkylene, optionally substituted with one or more substituents R¹¹;
A is C₁-C₆ 1-alkanyl-ylidene, optionally substituted with one or more substituents R¹¹;
R¹¹ is independently selected from the group consisting of H, branched or straight-chain C₂-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
Y is selected from the group consisting of C₁-C₆ alkylene, C₂-C₆ alkenylene, and C₂-C₆ alkynylene, wherein Y is optionally substituted with one or more substituents R²;
R² is independently selected from the group consisting of branched or straight-chain C₁-C₆ alkyl, branched or straight-chain C₂-C₆ alkenyl, branched or straight-chain C₂-C₆ alkynyl, hydroxy, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
R³ is H, branched or straight-chain C₁-C₆ alkyl, branched or straight-chain C₂-C₆ alkenyl, branched or straight-chain C₂-C₆ alkynyl, C₆-C₁₄ aryl or C₅-C₁₄ heteroaryl containing one or more heteroatoms selected from the group consisting of N, N(R⁷), O, S, and S(O)ₘ, wherein m is 1 or 2, and wherein R³, is optionally substituted with one or more substituents R⁸;
R⁷ is selected from the group consisting of H, branched or straight-chain C₁-C₆ alkyl, branched or straight-chain C₂-C₆ alkenyl, and branched or straight-chain C₂-C₆ alkynyl, wherein one or more hydrogens in the alkyl, alkenyl or alkynyl chain may be replaced by one or more fluorines;
R⁸ is independently selected from the group consisting of branched or straight-chain C₁-C₆ alkyl, branched or straight-chain C₂-C₆ alkenyl, branched or straight-chain C₂-C₆ alkynyl, hydroxy, NH₂, NH(C₁-C₆ alkyl), N(C₁-C₆ alkyl)₂, C₁-C₆ alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens in the alkyl, alkenyl or alkynyl chain may be replaced by one or more fluorines;
R⁹ is selected from the group consisting of CH₃S(O)₂-O-, CF₃S(O)₂-O-, CH₃(C₆H₄)S(O)₂-O-, Cl, Br, and I;
R¹² is selected from the group consisting of H, branched or straight-chain C₁-C₆ alkyl, branched or straight-chain C₂-C₆ alkenyl, branched or straight-chain C₂-C₆ alkynyl, hydroxy, C₁-C₆alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens can be replaced by one or more fluorines;
R¹³ is selected from the group consisting of H, branched or straight-chain C₁-C₆ alkyl, branched or straight-chain C₂-C₆ alkenyl, branched or straight-chain C₂-C₆ alkynyl, hydroxy, C₁-C₆alkoxy, CO₂H, CO₂(C₁-C₆ alkyl), CONH₂, CONH(C₁-C₆ alkyl), CON(C₁-C₆ alkyl)₂, nitro, cyano, and halo, wherein one or more hydrogens can be replaced by one or more fluorines; and
n is an integer from 1 to 1,500.

10. The method of claim 9, wherein W is methylene.

11. The method of claim 9, wherein A is methylidene.

12. The method of claim 9, wherein the activation step (a) comprises reacting the compound of the formula (IV) with methanesulfonyl chloride and a base.

13. The method of claim 9, wherein the oxidizing agent comprises hydrogen peroxide.

14. The method of claim 9, wherein the dehydrating step (d) comprises reacting the compound of formula (VII) with thionyl chloride and a base.

15. The method of claim 9, wherein the dehydrating step (d) comprises reacting the compound of formula (VII) with methane sulfonyl chloride and a base.

16. The method of claim 9, wherein n is an integer from 5 to 1,000.

17. The method of claim 16, wherein n is an integer from 20 to 500.

18. The method of claim 17, wherein n is an integer from 50 to 250.

19. The method of claim 9, wherein R³ is H.

20. The method of claim 9, wherein R³ is methyl.

21. The method of claim 9, wherein R³ is selected from the group consisting of phenyl, pyridyl, pyrimidinyl, and naphthyl.

22. The method of claim 21, wherein R³ is phenyl.

23. The method of claim 22, wherein R³ is phenyl and R⁸ is nitro.

## Patentansprüche

1. Ein Reagens, umfassend eine Verbindung der Formel (I): 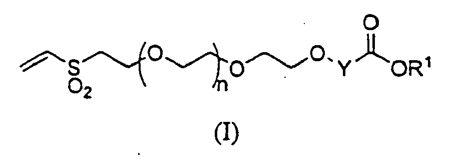 wobei:
Y aus der Gruppe bestehend aus C₁-C₆-Alkylen, C₂-C₆-Alkenylen und C₂-C₆-Alkinylen ausgewählt ist, wobei Y gegebenenfalls mit einem oder mehreren Substituenten R² substituiert ist;
R² unabhängig aus der Gruppe bestehend aus verzweigtem oder geradkettigem C₁-C₆-Alkyl, verzweigtem oder geradkettigem C₂-C₆-Alkenyl, verzweigtem oder geradkettigem C₂-C₆-Alkynyl, Hydroxy, C₁-C₆-Alkoxy, CO₂H, CO₂(C₁-C₆-Alkyl), CONH₂, CONH(C₁-C₆-Alkyl), CON(C₁-C₆-Alkyl)₂, Nitro, Cyano und Halogen ausgewählt ist, wobei ein oder mehrere Wasserstoffatome in der Alkyl-, Alkenyl- oder Alkinylkette durch ein oder
mehrere Fluoratome ersetzt sein können;
R¹ C₆-C₁₄-Aryl oder C₅-C₁₄-Heteroaryl, das ein oder mehrere Heteroatome, ausgewählt aus der Gruppe bestehend aus N, N(R⁷), O, S und S(O)ₘ, enthält, ist, wobei m 1 oder 2 ist und wobei R¹ gegebenenfalls mit einem oder mehreren Substituenten R⁸ substituiert ist;
R⁷ aus der Gruppe bestehend aus H, verzweigtem oder geradkettigem C₁-C₆-Alkyl,
verzweigtem oder geradkettigem C₂-C₆-Alkenyl und verzweigtem oder geradkettigem C₂-C₆-Alkinyl ausgewählt ist, wobei ein oder mehrere Wasserstoffatome in der Alkyl-,
Alkenyl- oder Alkinylkette durch ein oder mehrere Fluoratome ersetzt sein können;
R⁸ unabhängig aus der Gruppe bestehend aus verzweigtem oder geradkettigem C₁-C₆-Alkyl, verzweigtem oder geradkettigem C₂-C₆-Alkenyl, verzweigtem oder geradkettigem C₂-C₆-Alkinyl, Hydroxy, NH₂, NH(C₁-C₆-Alkyl), N(C₁-C₆-Alkyl)₂, C₁-C₆-Alkoxy, CO₂H, CO₂(C₁-C₆-Alkyl), CONH₂, CONH(C₁-C₆-Alkyl), CON(C₁-C₆-Alkyl)₂, Nitro,
Cyano und Halogen ausgewählt ist, wobei ein oder mehrere Wasserstoffatome in der Alkyl-, Alkenyl- oder Alkinylkette durch ein oder mehrere Fluoratome ersetzt sein können; und
n eine ganze Zahl von 1 bis 1.500 ist.

2. Das Reagens nach Anspruch 1, wobei n eine ganze Zahl von 5 bis 1.000 ist.

3. Das Reagens nach Anspruch 2, wobei n eine ganze Zahl von 20 bis 500 ist.

4. Das Reagens nach Anspruch 3, wobei n eine ganze Zahl von 50 bis 250 ist.

5. Das Reagens nach Anspruch 1, wobei Y Methylen ist.

6. Das Reagens nach Anspruch 1, wobei R¹ aus der Gruppe bestehend aus Phenyl, Pyridyl, Pyrimidinyl und Naphthyl ausgewählt ist.

7. Das Reagens nach Anspruch 1, wobei R¹ Phenyl ist.

8. Das Reagens nach Anspruch 1, wobei R¹ Phenyl ist und R⁸ Nitro ist.

9. Ein Verfahren zur Herstellung einer Verbindung der Formel (VIII) 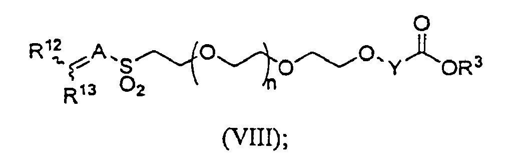 umfassend die Schritte:
(a) Umsetzen einer Verbindung der Formel (IV): 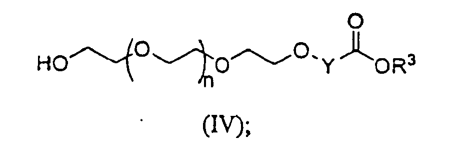 mit einem aktivierenden Rest, um eine Verbindung der Formel (IVa) zu bilden: 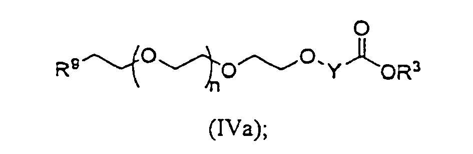
(b) Umsetzen der Verbindung der Formel (IVa) mit einer Verbindung der Formel (V): 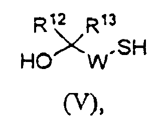 um eine Verbindung der Formel (VI) zu bilden: 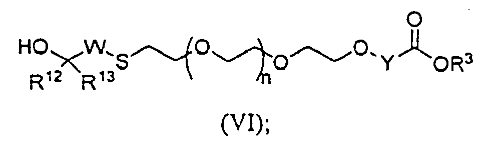
(c) Oxidieren der Verbindung der Formel (VI), um eine Verbindung der Formel (VII) zu bilden: 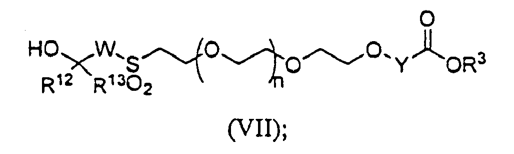 und
(d) Dehydratisieren der Verbindung der Formel (VII), um eine Verbindung der Formel (VIII) zu bilden,
wobei:
W C₁-C₆-Alkylen ist, das gegebenenfalls mit einem oder mehreren Substituenten R¹¹ substituiert ist;
A C₁-C₆-1-Alkanyl-yliden ist, das gegebenenfalls mit einem oder mehreren Substituenten R¹¹ substituiert ist;
R¹¹ unabhängig aus der Gruppe bestehend aus H, verzweigtem oder geradkettigem C₁-C₆-Alkyl, verzweigtem oder geradkettigem C₂-C₆-Alkenyl, verzweigtem oder
geradkettigem C₂-C₆-Alkinyl, Hydroxy, C₁-C₆-Alkoxy, CO₂H, CO₂(C₁-C₆-Alkyl), CONH₂, CONH(C₁-C₆-Alkyl), CON(C₁-C₆-Alkyl)₂, Nitro, Cyano und Halogen ausgewählt ist, wobei ein oder mehrere Wasserstoffatome in der Alkyl-, Alkenyl- oder Alkinylkette durch ein oder mehrere Fluoratome ersetzt sein können;
Y aus der Gruppe bestehend aus C₁-C₆-Alkylen, C₂-C₆-Alkenylen und C₂-C₆-Alkinylen ausgewählt ist, wobei Y gegebenenfalls mit einem oder mehreren Substituenten R² substituiert ist;
R² unabhängig aus der Gruppe bestehend aus verzweigtem oder geradkettigem C₁-C₆-Alkyl, verzweigtem oder geradkettigem C₂-C₆-Alkenyl, verzweigtem oder geradkettigem C₂-C₆-Alkinyl, Hydroxy, C₁-C₆-Alkoxy, CO₂H, CO₂(C₁-C₆-Alkyl), CONH₂, CONH(C₁-C₆-Alkyl), CON(C₁-C₆-Alkyl)₂, Nitro, Cyano und Halogen ausgewählt ist, wobei ein oder mehrere Wasserstoffatome in der Alkyl-, Alkenyl- oder Alkinylkette durch ein oder
mehrere Fluoratome ersetzt sein können;
R³ H, verzweigtes oder geradkettiges C₁-C₆-Alkyl, verzweigtes oder geradkettiges C₂-C₆-Alkenyl, verzweigtes oder geradkettiges C₂-C₆-Alkinyl, C₆-C₁₄-Aryl oder C₅-C₁₄-Heteroaryl, das ein oder mehrere Heteroatome, ausgewählt aus der Gruppe bestehend aus N, N(R⁷), O, S und S(O)ₘ, enthält, ist, wobei m 1 oder 2 ist und wobei R³ gegebenenfalls mit einem oder mehreren Substituenten R⁸ substituiert ist;
R⁷ aus der Gruppe bestehend aus H, verzweigtem oder geradkettigem C₁-C₆-Alkyl,
verzweigtem oder geradkettigem C₂-C₆-Alkenyl und verzweigtem oder geradkettigem C₂-C₆-Alkinyl ausgewählt ist, wobei ein oder mehrere Wasserstoffatome in der Alkyl-,
Alkenyl- oder Alkinylkette durch ein oder mehrere Fluoratome ersetzt sein können;
R⁸ unabhängig aus der Gruppe bestehend aus verzweigtem oder geradkettigem C₁-C₆-Alkyl, verzweigtem oder geradkettigem C₂-C₆-Alkenyl, verzweigtem oder geradkettigem C₂-C₆-Alkinyl, Hydroxy, NH₂, NH(C₁-C₆-Alkyl), N(C₁-C₆-Alkyl)₂, C₁-C₆-Alkoxy, CO₂H, CO₂(C₁-C₆-Alkyl), CONH₂, CONH(C₁-C₆-Alkyl), CON(C₁-C₆-Alkyl)₂, Nitro,
Cyano und Halogen ausgewählt ist, wobei ein oder mehrere Wasserstoffatome in der Alkyl-, Alkenyl- oder Alkinylkette durch ein oder mehrere Fluoratome ersetzt sein können;
R⁹ aus der Gruppe bestehend aus CH₃S(O)₂-O-, CF₃S(O)₂-O-, CH₃(C₆H₄)S(O)₂-O-, Cl,
Br und I ausgewählt ist;
R¹² aus der Gruppe bestehend aus H, verzweigtem oder geradkettigem C₁-C₆-Alkyl,
verzweigtem oder geradkettigem C₂-C₆-Alkenyl, verzweigtem oder geradkettigem C₂-C₆-Alkinyl, Hydroxy, C₁-C₆-Alkoxy, CO₂H, CO₂(C₁-C₆-Alkyl), CONH₂, CONH(C₁-C₆-Alkyl), CON(C₁-C₆-Alkyl)₂, Nitro, Cyano und Halogen ausgewählt ist, wobei ein oder mehrere Wasserstoffatome durch ein oder mehrere Fluoratome ersetzt sein können;
R¹³ aus der Gruppe bestehend aus H, verzweigtem oder geradkettigem C₁-C₆-Alkyl, verzweigtem oder geradkettigem C₂-C₆-Alkenyl, verzweigtem oder geradkettigem C₂-C₆-Alkinyl, Hydroxy, C₁-C₆-Alkoxy, CO₂H, CO₂(C₁-C₆-Alkyl), CONH₂, CONH(C₁-C₆-Alkyl), CON(C₁-C₆-Alkyl)₂, Nitro, Cyano und Halogen ausgewählt ist, wobei ein oder mehrere Wasserstoffatome durch ein oder mehrere Fluoratome ersetzt sein können; und n eine ganze Zahl von 1 bis 1.500 ist.

10. Das Verfahren nach Anspruch 9, wobei W Methylen ist.

11. Das Verfahren nach Anspruch 9, wobei A Methyliden ist.

12. Das Verfahren nach Anspruch 9, wobei der Aktivierungsschritt (a) Umsetzen der Verbindung der Formel (IV) mit Methansulfonylchlorid und einer Base umfasst.

13. Das Verfahren nach Anspruch 9, wobei das Oxidationsmittel Wasserstoffperoxid umfasst.

14. Das Verfahren nach Anspruch 9, wobei der Dehydratisierungsschritt (d) Umsetzen der Verbindung der Formel (VII) mit Thionylchlorid und einer Base umfasst.

15. Das Verfahren nach Anspruch 9, wobei der Dehydratisierungsschritt (d) Umsetzen der Verbindung der Formel (VII) mit Methansulfonylchlorid und einer Base umfasst.

16. Das Verfahren nach Anspruch 9, wobei n eine ganze Zahl von 5 bis 1.000 ist.

17. Das Verfahren nach Anspruch 16, wobei n eine ganze Zahl von 20 bis 500 ist.

18. Das Verfahren nach Anspruch 17, wobei n eine ganze Zahl von 50 bis 250 ist.

19. Das Verfahren nach Anspruch 9, wobei R³ H ist.

20. Das Verfahren nach Anspruch 9, wobei R³ Methyl ist.

21. Das Verfahren nach Anspruch 9, wobei R³ aus der Gruppe bestehend aus Phenyl, Pyridyl, Pyrimidinyl und Naphthyl ausgewählt ist.

22. Das Verfahren nach Anspruch 21, wobei R³ Phenyl ist.

23. Das Verfahren nach Anspruch 22, wobei R³ Phenyl ist und R⁸ Nitro ist.

## Revendications

1. Réactif comprenant un composé de la formule (I): 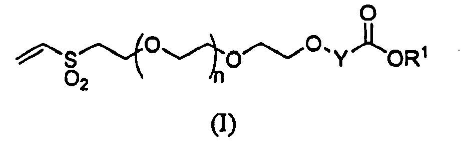 où:
Y est sélectionné dans le groupe consistant en alkylèneC₁-C₆, alkénylèneC₂-C₆ et alkynylèneC₂-C₆, où Y est optionnellement substitué par un ou plusieurs substituants R²;
R² est indépendamment sélectionné dans le groupe consistant en alkyleC₁-C₆ à chaîne ramifiée ou droite, alkényleC₂-C₆ à chaîne ramifiée ou droite, alkynyleC₂-C₆ à chaîne ramifiée ou droite, hydroxy, alcoxyC₁-C₆, CO₂H, CO₂(alkyleC₁-C₆), CONH₂, CONH(alkyleC₁-C₆), CON(alkyleC₁-C₆)₂, nitro, cyano et halo, où un ou plusieurs hydrogènes dans la chaîne alkyle, alkényle ou alkynyle peuvent être remplacés par un ou plusieurs fluors;
R¹ est aryleC₆-C₁₄, ou hétéroaryleC₅-C₁₄ contenant un ou plusieurs hétéroatomes sélectionnés dans le groupe consistant en N, N(R⁷), O, S et S(O)ₘ, où m est 1 ou 2, et où R¹ est optionnellement substitué par un ou plusieurs substituants R⁸;
R⁷ est sélectionné dans le groupe consistant en H, alkyleC₁-C₆ à chaîne ramifiée ou droite, alkényleC₂-C₆ à chaîne ramifiée ou droite et alkynyleC₂-C₆ à chaîne ramifiée ou droite, où un ou plusieurs hydrogènes dans la chaîne alkyle, alkényle ou alkynyle peuvent être remplacés par un ou plusieurs fluors;
R⁸ est indépendamment sélectionné dans le groupe consistant en alkyleC₁-C₆ à chaîne ramifiée ou droite, alkényleC₂-C₆ à chaîne ramifiée ou droite, alkynyleC₂-C₆ à chaîne ramifiée ou droite, hydroxy, NH₂, NH(alkyleC₁-C₆), N(alkyleC₁-C₆)₂, alkoxyC₁-C₆, CO₂H, CO₂(alkyleC₁-C₆), CONH₂, CONH(alkyleC₁-C₆), CON(alkyleC₁-C₆)₂, nitro, cyano, et halo, où un ou plusieurs hydrogènes dans la chaîne alkyle, alkényle ou alknyle peuvent être remplacés par un ou plusieurs fluors; et
n est un entier de 1 à 1500.

2. Réactif selon la revendication 1, où n est un entier de 5 à 1000.

3. Réactif selon la revendication 2, où n est un entier de 20 à 500.

4. Réactif selon la revendication 3, où n est un entier de 50 à 250.

5. Réactif selon la revendication 1, où Y est méthylène.

6. Réactif selon la revendication 1, où R¹ est sélectionné dans le groupe consistant en phenyle, pyridyle, pyrimidinyle et naphtyle.

7. Réactif selon la revendication 1, où R¹ est phényle.

8. Réactif selon la revendication 1, où R¹ est phényle et R⁸ est nitro.

9. Méthode de production d'un composé de la formule (VIII), 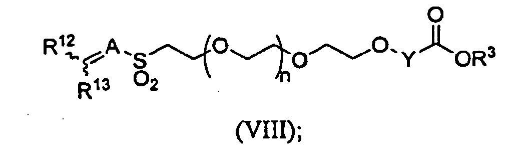 comprenant les étapes de:
(a) faire réagir un composé de la formule (IV): 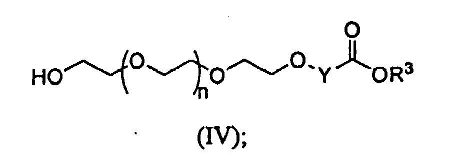 avec un groupe d'activation pour former un composé de la formule (IVa): 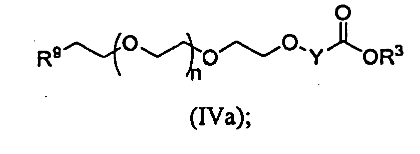
(b) faire réagir le composé de la formule (IVa) avec un composé de la formule (V): 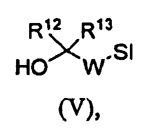 pour former un composé de la formule (VI) : 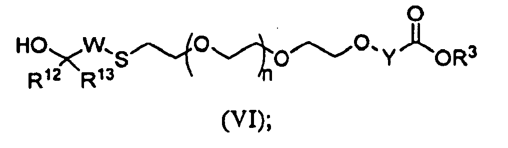
(c) oxyder le composé de la formule (VI) pour former un composé de la formule (VII): 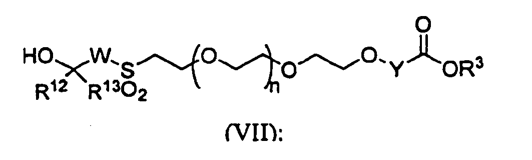
et (d) déshydrater le composé de la formule (VII) pour former un composé de la formule (VIII),
où:
W est alkylèneC₁-C₆, optionnellement substitué par un ou plusieurs substituants R¹¹;
A est 1-alkanyl-ylidèneC₁-C₆, optionnellement substitué par un ou plusieurs substituants R¹¹;
R¹¹ est indépendamment sélectionné dans le groupe consistant en H, alkyleC₁-C₆ à chaîne ramifiée ou droite, alkényleC₂-C₆ à chaîne ramifiée ou droite, alkynyleC₂-C₆ à chaîne ramifiée ou droite, hydroxy, alcoxyC₁-C₆, CO₂H, CO₂(alkyleC₁-C₆), CONH₂, CONH(alkyleC₁-C₆), CON(alkyleC₁-C₆)₂, nitro, cyano et halo, où un ou plusieurs hydrogènes dans la chaîne alkyle, alkényle ou alkynyle peuvent être remplacés par un ou plusieurs fluors;
Y est sélectionné dans le groupe consistant en alkylèneC₁-C₆, alkénylèneC₂-C₆ et alkynylèneC₂-C₆, où Y est optionnellement substitué par un ou plusieurs substituants R²;
R² est indépendamment sélectionné dans le groupe consistant en alkyleC₁-C₆ à chaîne ramifiée ou droite, alkényleC₂-C₆ à chaîne ramifiée ou droite, alkynyleC₂-C₆ à chaîne ramifiée ou droite, hydroxy, alcoxyC₁-C₆, CO₂H, CO₂(alkyleC₁-C₆), CONH₂, CONH(alkyleC₁-C₆), CON( alkyleC₁-C₆)₂, nitro, cyano et halo, où un ou plusieurs hydrogènes dans la chaîne alkyle, alkényle ou alkynyle peuvent être remplacés par un ou plusieurs fluors;
R³ est H, alkyleC₁-C₆ à chaîne ramifiée ou droite, alkényleC₂-C₆ à chaîne ramifiée ou droite, alkynyleC₂-C₆ à chaîne ramifiée ou droite, aryleC₆-C₁₄ ou hétéroaryleC₅-C₁₄ contenant un ou plusieurs hétéroatomes sélectionnés dans le groupe consistant en N, N(R⁷), O, S et S(O)ₘ, où m est 1 ou 2, et où R³ est optionnellement substitué par un ou plusieurs substituants R⁸;
R⁷ est sélectionné dans le groupe consistant en H, alkyleC₁-C₆ à chaîne ramifiée ou droite, alkényleC₂-C₆ à chaîne ramifiée ou droite et alkynyleC₂-C₆ à chaîne ramifiée ou droite, où un ou plusieurs hydrogènes dans la chaîne alkyle, alkényle ou alkynyle peuvent être remplacés par un ou plusieurs fluors;
R⁸ est indépendamment sélectionné dans le groupe consistant en alkyleC₁-C₆ à chaîne ramifiée ou droite, alkényleC₂-C₆ à chaîne ramifiée ou droite, alkynyleC₂-C₆ à chaîne ramifiée ou droite, hydroxy, NH₂, NH(alkyleC₁-C₆), N(alkyleC₁-C₆)₂, alcoxyC₁-C₆, CO₂H, CO₂(alkyleC₁-C₆), CONH₂, CONH(alkyleC₁-C₆), CON(alkyleC₁-C₆)₂, nitro, cyano et halo, où un ou plusieurs hydrogènes dans la chaîne alkyle, alkényle ou alkynyle peuvent être remplacés par un ou plusieurs fluors;
R⁹ est sélectionné dans le groupe consistant en CH₃S(O)₂-O-, CF₃S(O)₂-O-, CH₃(C₆H₄)S(O)₂-O-, Cl, Br et I;
R¹² est sélectionné dans le groupe consistant en H, alkyleC₁-C₆ à chaîne ramifiée ou droite, alkényleC₂-C₆ à chaîne ramifiée ou droite, alkynyleC₂-C₆ à chaîne ramifiée ou droite, hydroxy, alcoxyC₁-C₆, CO₂H, CO₂(alkyleC₁-C₆), CONH₂, CONH(alkyleC₁-C6), CON(alkyleC₁-C₆)₂, nitro, cyano et halo, où un ou plusieurs hydrogènes peuvent être remplacés par un ou plusieurs fluors;
R¹³ est sélectionné dans le groupe consistant en H, alkyleC₁-C₆ à chaîne ramifiée ou droite, alkényleC₂-C₆ à chaîne ramifiée ou droite, alkynyleC₂-C₆ à chaîne ramifiée ou droite, hydroxy, alcoxyC₁-C₆, CO₂H, CO₂(alkyleC₁-C₆), CONH₂, CONH(alkyleC₁-C₆), CON(alkyleC₁-C₆)₂, nitro, cyano et halo, où un ou plusieurs hydrogènes peuvent être remplacés par un ou plusieurs fluors; et
n est un entier de 1 à 1500.

10. Méthode selon la revendication 9, où W est méthylène.

11. Méthode selon la revendication 9, où A est méthylidène.

12. Méthode selon la revendication 9, où l'étape d'activation (a) comprend la reaction du composé de la formule (IV) avec du méthanesulfonyl chlorure et une base.

13. Méthode selon la revendication 9, où l'agent d'oxydation comprend le péroxyde d'hydrogène.

14. Méthode selon la revendication 9, où l'étape de déshydratation (d) comprend la reaction du composé de la formule (VII) avec du thionyl chlorure et une base.

15. Méthode selon la revendication 9, dans laquelle l'étape de déshydratation (d) comprend la reaction du composé de la formule (VII) avec du méthane sulfonyl chlorure et une base.

16. Méthode selon la revendication 9, où n est un entier de 5 à 1000.

17. Méthode selon la revendication 16, où n est un entier de 20 à 500.

18. Méthode selon la revendication 17, où n est un entier de 50 à 250.

19. Méthode selon la revendication 9, où R³ est H.

20. Méthode selon la revendication 9, où R³ est méthyle.

21. Méthode selon la revendication 9, où R³ est sélectionné dans le groupe consistant en phényle, pyridyle, pyrimidinyle et naphtyle.

22. Méthode selon la revendication 21, où R³ est phényle.

23. Méthode selon la revendication 22, où R³ est phényle et R⁸ est nitro.
